Europäisches Patentamt

European Patent Office

Office européen des brevets

(11) Veröffentlichungsnummer: **0 003 987**
**B1**

(12) # EUROPÄISCHE PATENTSCHRIFT

(21) Anmeldenummer: **79100474.0**

(22) Anmeldetag: **19.02.79**

(51) Int. Cl.³: **C 07 D 307/32**

(54) Verfahren zur Extraktion von Pantolacton aus seinen wässrigen Lösungen

(30) Priorität: **03.03.78 DE 2809179**

(43) Veröffentlichungstag der Anmeldung:
**19.09.79 Patentblatt 79/19**

(45) Bekanntmachung des Hinweises auf die Patenterteilung:
**12.11.80 Patentblatt 80/23**

(84) Benannte Vertragsstaaten:
**CH DE FR GB IT**

(56) Entgegenhaltungen:
**FR - A - 871 075**

(73) Patentinhaber: **BASF Aktiengesellschaft**
**Carl-Bosch-Strasse 38**
**D - 6700 Ludwigshafen (DE)**

(72) Erfinder: **Paust, Joachim, Dr.**
**Ringstrasse 3**
**D - 6701 Neuhofen (DE)**
**Schmidt, Wolfram, Dr.**
**Im Rosengarten Ost 8**
**D - 6701 Friedelsheim (DE)**

Verfahren zur Extraktion von Pantolacton aus seinen wäßrigen Lösungen

Die vorliegende Erfindung betrifft ein verbessertes Verfahren zur Extraktion von Pantolacton aus seinen wäßrigen Lösungen.

Pantolacton (das Lacton der 2,4 - Dihydroxy - 3,3 - dimethyl - buttersäure) ist ein Zwischenprodukt bei der Herstellung von Pantothensäure und verwandten physiologisch aktiven Verbindungen. Nach den technisch gebräuchlichen Synthesen erhält man es durch Hydrolyse des 2,4 - Dihydroxy - 3,3 - butyronitrils, wobei es in Form saurer wäßriger Lösungen anfällt, aus denen es isoliert werden muß. Hierzu wendet man Extraktionsverfahren an, jedoch lassen die bisher üblichen Extraktionsmittel zu wünschen übrig. Die für diesen Zweck hauptsächlich versendeten chlorierten Kohlenwasserstoffe wie Chloroform und Methylenchlorid (vgl. z.B. DE-AS 22 28 641) sind zwar, betrachtet man sie lediglich unter dem Aspekt der Extraktion, gut geeignet, weisen aber für technische Synthesen wegen ihrer Toxizität aus Umweltgründen erheblich Nachteile auf. Infolge ihres hohen Dampfdruckes gelangen sie stets in die Abluft, aus der sie nur mit hohem Aufwand entfernt werden können. Auch Diäthyläther wurde bereits als Extraktionsmittel verwendet (DE-AS 15 68 755), jedoch erfordert dieser wegen seines relativ geringen Lösevermögens für das Pantolacton wirtschaftlich nicht tolerierbare Mengen des Extraktionsmittels. Für großtechnische Verfahren ist Diäthyläther auch deshalb schlecht geeignet, weil er sich merklich in Wasser löst und weil er, wie die meisten anderen Äther, zur Bildung von Peroxiden neigt.

Der Erfindung lag daher die Aufgabe zugrunde, Pantolacton aus seinen wäßrigen Lösungen durch Wahl eines besser geeigneten Extraktionsmittels auf insgesamt wirtschaftlichere Weise zu isolieren.

Es wurde gefunden, daß man Pantolacton mit Hilfe eines organischen Extraktionsmittels aus seinen wäßrigen Lösungen mit gutem Erfolg extrahieren kann, wenn man hierzu als Extraktionsmittel den Methyl - tert. - butyläther (MTB) verwendet.

Hinsichtlich des Verteilungsquotienten ist der MTB für die Extraktion des Pantolactons aus wäßriger Phase so gut geeignet wie Methylenchlorid, ohne jedoch die Nachteile des chlorierten Kohlenwasserstoffs zu haben. Der in die Abluft gelangende MTB kann schadlos verbrannt werden, und im Abwasser ist er biologisch abbaubar. Weiterhin bildet MTB keine Peroxide und ferner ist er aus den billigen Ausgangsstoffen Methanol und Isobuten leicht zugänglich. Schließlich bietet der MTB in verfahrenstechnischer Hinsicht den Vorteil, daß die MTB-Phase stets wesentlich leichter bleibt als die wäßrige Phase, so daß die Phasentrennung keine Schwierigkeiten bereitet, wie es für Methylenchlorid zutrifft, welches etwa ebenso schwer ist wie die wäßrige Phase.

Bei den zu extrahierenden Lösungen handelt es sich zumeist um saure wäßrige Lösungen mit einem Anfangs - Pantolactongehalt von 6 bis 20 Gew.%. Pro Kilogramm einer solchen Lösung verwendet man zweckmäßigerweise 0,5 bis 2 kg MTB, um pro Extraktionsschritt eine Abreicherung auf etwa jeweils 20% der ursprünglichen Pantolactonkonzentration in der wäßrigen Phase zu erzielen. Im übrigen kann man die Extraktion nach allen hierfür bekannten Techniken vornehmen, also diskontinuierlich, in Kaskadenschaltung oder vollkontinuierlich im flüssig - flüssig - Gegenstromverfahren unter Verwendung von Füllkörper- oder Bodenkolonnen bei Normaldruck oder leicht erhöhtem Druck und vorzugsweise bei 20 bis 40°C. Das erfindungsgemäße Verfahren ist sowohl bei der Isolierung von D,L-Pantolacton aus seinem wäßrigen Synthesegemisch als auch zur Isolierung der reinen optischen Isomeren nach deren Racematspaltung über Pantoesäuresalze und Rückbildung des D- bzw. L-Pantolactons geeignet, und zwar unabhängig davon, ob die wäßrige Lösung noch weitere Stoffe wie vornehmlich Salze, z.B. Ammoniumsulfat und Natriumsulfat, enthält. Die Gegenwart von Salzen ist wegen des Aussalzeffektes sogar von Vorteil.

Auch die Aufarbeitung der MTB - Pantolacton - Lösungen erfolgt nach an sich bekannten Methoden, z.B. durch Destillation oder, im Falle von weitgehend reinen optischen Pantolacton - Isomeren, vorzugsweise durch Kristallisation. Für manche Zwecke lassen sich die Lösungen auch unmittelbar weiterverwenden.

### Beispiel

Eine von der Synthese des Pantolactons her stammende wäßrige Lösung, die 22 Gew.% (wie im folgenden) Pantolacton, 3% Schwefelsäure, 11% Ammonsulfat und 12% Natriumsulfat enthielt, wurde 10 Minuten lang bei 25°C mit 0,6 kg MTB pro Kilogramm der Lösung gerührt und nach der Phasentrennung destillativ auf das Pantolacton aufgearbeitet.

90% des Pantolactons wurden hierbei gewonnen, der Rest verblieb in der wäßrigen Phase, aus welcher mit je weiteren 0,6 kg MTB das restliche Pantolacton in zwei Folgeschritten praktisch quantitativ extrahiert wurde. Die Verluste an MTB betrugen nur 1% der insgesamt eingesetzten Menge.

### Patentanspruch

Verfahren zur Extraktion von Pantolacton aus seinen wäßrigen Lösungen mit Hilfe eines organischen Extraktionsmittels, *dadurch gekennzeichnet,* daß man als Extraktionsmittel den Methyl - tert. - butyläther verwendet.

## Claim

A process for the extraction of pantolactone from its aqueous solutions by means of an organic extractant, *characterised in that* methyl tert.-butyl ether is used as the extractant.

## Revendication

Procédé pour l'extraction de pantolactone à partir de ses solutions aqueuses à l'aide d'un agent d'extraction organique, caractérisé en ce qu'on utilise, en tant qu'agent d'extraction, le méthyl – tertiobutyléther.